# EUROPÄISCHE PATENTANMELDUNG

(11) **EP 0 941 988 A2**
(43) Veröffentlichungstag der Anmeldung: **15.09.1999**
(21) Anmeldenummer: 99104435.5
(22) Anmeldetag: 05.03.1999
(51) Int. Cl.: C07C 259/18, C07C 257/20

(54) **Neue Benzamidoxim-Derivate, Zwischenprodukte und Verfahren zu deren Herstellung und deren Verwendung als Fungizide**

(30) Priorität: 10.03.1998 DE 19810142
(71) Anmelder: BASF AKTIENGESELLSCHAFT, 67056 Ludwigshafen (DE)
(72) Erfinder: Eicken, Karl, Dr., 67157 Wachenheim (DE); Rheinheimer, Joachim, Dr., 67063 Ludwigshafen (DE); Rose, Ingo, Dr., 68159 Mannheim (DE); Rack, Michael, Dr., 69123 Heidelberg (DE); Ammermann, Eberhard, Dr., 64646 Heppenheim (DE); Lorenz, Gisela, Dr., 67434 Neustadt (DE); Strathmann, Siegfried, Dr., 67117 Limburgerhof (DE)

(57) **Zusammenfassung**

Benzamidoxim-Derivate der Formel I 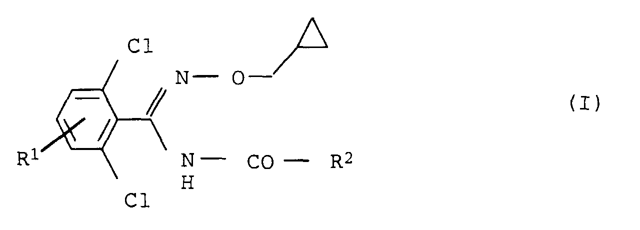 in der die Substituenten die folgende Bedeutung haben:
- R¹: Fluor
- R²: Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches ggf. einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazol-C₁-C₄-Alkyl, welches ggf. einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann.

## Beschreibung

Die vorliegende Erfindung betrifft neue Benzamidoxim-Derivate, Verfahren und Zwischenprodukte zu deren Herstellung und deren Verwendung als Fungizide.

Aus der JP-A 02/006453 sind Benzamidoxim-Derivate mit fungizider Wirksamkeit beschrieben, die jedoch insbesondere bei niedrigen Aufwandmengen nicht in vollem Umfang zufrieden stellen können.

Aufgabe der vorliegenden Erfindung war es daher, neue Benzamidoxim-Derivate mit verbesserter Wirkung, insbesondere auch bei niedrigen Aufwandmengen, zur Verfügung zu stellen.

Überraschenderweise wurde nun gefunden, daß Benzamidoxim-Derivate der Formel I 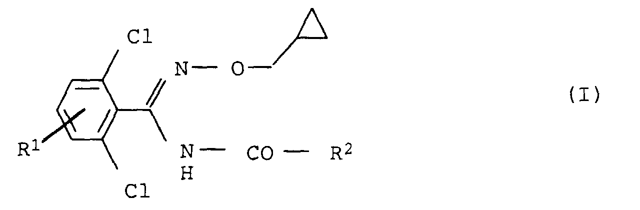 wobei die Substituenten die folgenden Bedeutungen haben:
- R¹: Fluor
- R²: Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches ggf. einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazol-C₁-C₄-Alkyl, welches ggf. einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann.

Bei der Definition der Substituenten R₁und R₂ stehen die angegebenen Begriffe als SAmmelbegriff für eine Gruppe von Verbindungen.

Die Bedeutung Halogen steht jeweils für Fluor, Brom, Chlor oder Iod, insbesondere für Fluor oder Chlor.

Ferner stehen beispielsweise:
- C₁-C₄-Alkyl für: Methyl, Ethyl, n-Propyl, 1-Methylethyl, n-Butyl, 1-Methylpropyl, 2-Methylpropyl oder 1,1-Dimethylethyl, insbesondere für Methyl oder Ethyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, insbesondere für Trifluormethyl;
- C₁-C₄-Alkoxy für: Methoxy, Ethoxy, n-Propoxy, 1-Methylethoxy, n-Butoxy, 1-Methylpropoxy, 2-Methylpropoxy oder 1,1-Dimethylethoxy, insbesondere für Methoxy oder Ethoxy;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, insbesondere für Difluormethoxy;
- Phenyl-C₁-C₆-alkyl für: z.B. Benzyl, 1-Phenylethyl, 2-Phenylethyl, 1-Phenylprop-1-yl, 2-Phenylprop-1-yl, 3-Phenylprop-1-yl, 1-(Phenylmethyl)-eth-1-yl, 1-(Phenylmethyl)-1-(methyl)-eth-1-yl oder 1-(Phenylmethyl)-prop-1-yl, insbesondere für Benzyl oder 2-Phenylethyl;
- Thienyl-C₁-C₄-alkyl für: z.B. 2-Thienylmethyl, 3-Thienylmethyl, 2-Thienylethyl, 2-Thienylprop-1-yl oder 3-Thienylprop-1-yl;
- Pyrazol-C₁-C₄-alkyl für: z.B. 1-Pyrazolyl-methyl, 2-pyrazolyl-methyl, 3-Pyrazolylmethyl, 2-Pyrazolylylethyl, 2-Pyrazolylylprop-1-yl oder 3-Pyrazolylprop-1-yl;

Verbindungen in denen der Substituent R² für Benzyl, welches am Phenylring einen bis drei Substituenten ausgewählt aus der vorstehend genannten Gruppe, insbesondere einen bis drei Substituenten ausgewählt aus Fluor, Chlor, Methyl, Methoxy oder Trifluormethyl, steht, haben sich als in der Regel besonders wirksam erwiesen. Insbesondere seien 4-Fluorbenzyl, 4-Chlorbenzyl, 4-Methylbenzyl, 4-Methoxybenzyl, 4-Trifluormethylbenzyl und 4-Difluormethoxybenzyl als besonders bevorzugte Substituenten R² erwähnt.

Verbindungen der Formel I, in denen R¹ und R² die in der nachstehenden Tabelle 1 aufgeführten Bedeutungen haben, sind insbesondere bevorzugt.

**Tabelle 1**

| **Nr.** | **R**^{**1**} | **R**^{**2**} |
|---|---|---|
| I.1 | 5-F | C₆H₅-CH₂ |
| I.2 | 5-F | 4-F-C₆H₄-CH₂ |
| I.3 | 5-F | 4-Cl-C₆H₄-CH₂ |
| I.4 | 5-F | 4-CH₃O-C₆H₄-CH₂ |
| I.5 | 5-F | 4-CF₃-C₆H₄-CH₂ |
| I.6 | 5-F | 4-CH₃-C₆H₄-CH₂ |
| I.7 | 5-F | 2-F-C₆H₄-CH₂ |
| I.8 | 5-F | 3-CH₃-C₆H₄-CH₂ |
| I.9 | 5-F | 2-Thienylmethyl |
| I.10 | 5-F | 3-Thienylmethyl |

Die erfindungsgemäßen Benzamidoxim-Derivate der Formel I erhält man nach dem erfindungsgemäßen Verfahren durch Umsetzung von Benzonitrilen der Formel II (in EP 713,862 beschrieben) mit Hydroxylamin oder dessen Salzen in wäßriger Lösung, vorzugsweise in Wasser oder WAsser/Alkanol-Gemischen, gegebenenfalls in Anwesenheit einer Base zu den Benzamidoximen der Formel III, die dann anschließend in an sich bekannter Weise in Gegenwart einer Base mit Cyclopropylmethylhalogenid zu den Verbindungen der Formel IV umgesetzt werden.

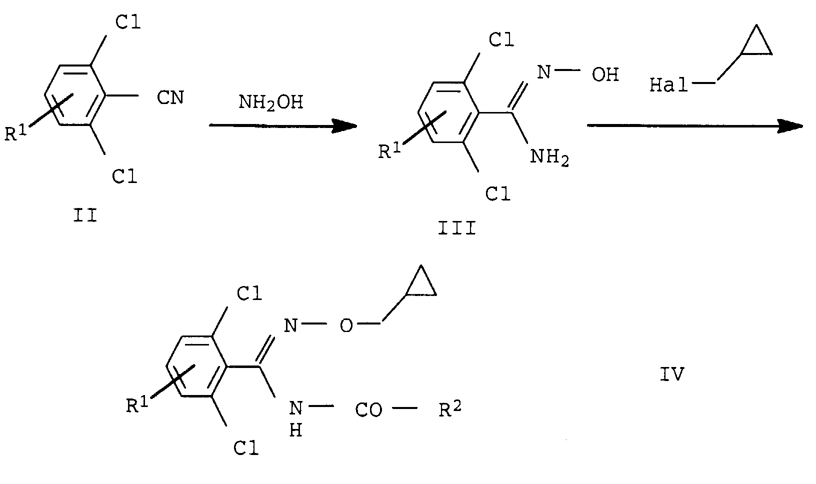

Ferner lassen sich die erfindungsgemäßen Benzamidoxim-Derivate der Formel III auch durch Umsetzung von Benzaldoximen der Formel V (in EP 713,862 beschrieben) mit Halogenierungsmitteln, insbesondere Chlor, in inerten Lösungsmitteln zu Benzhydroxamsäurehalogeniden der Formel VI und anschließende unmittelbare Umsetzung mit Ammoniak zu den Verbindungen III herstellen.

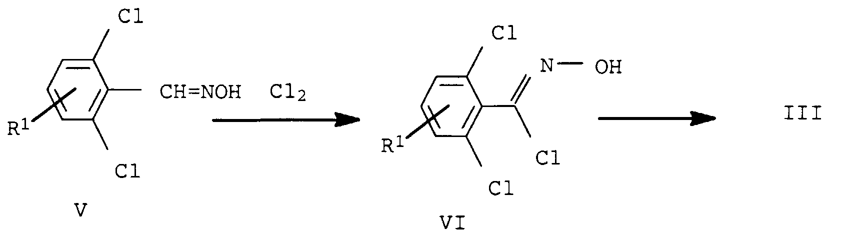

Die Benzamidoxime der Formel IV können dann in an sich bekannter Weise mit den entsprechenden Säurehalogeniden, vorzugsweise den entsprechenden Säurechloriden, durch Erwärmen in inerten Lösungsmitteln (vorzugsweise auf Temperaturen im Bereich von 20 bis 100 °C) acyliert werden.

Als inerte Lösungsmittel eignen sich insbesondere Kohlenwasserstoffe oder Ether, besonders bevorzugt aromatische Kohlenwasserstoffe wie Toluol oder Xylol, um nur zwei Beispiele zu nennen.

Die in vorstehendem Reaktionsschema aufgeführten Zwischenprodukte der Formel III und die Zwischenprodukte der Formel IV sind neu. Bevorzugte Verbindungen sind solche, in denen R¹ und R² den für die Verbindungen I genannten Bedeutungen entsprechen.

Die Verbindungen I zeichnen sich durch eine hervorragende Wirkung gegen ein breites Spektrum von pflanzenpathogenen Pilzen, insbesondere aus der Klasse der Ascomyceten, Deuteromyceten, Phycomyceten und Basidiomyceten, aus. Sie sind z.T. systemisch wirksam und können daher auch als Blatt- und Bodenfungizide eingesetzt werden.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-iso-butylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylen-diamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthal-säure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Di-hydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethyl-acetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-di-oxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethyl-amino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl]acetat, Methyl-E-2-{2-[6-(2-cyanophenoxy)pyridimin-4-yl-oxy]phenyl}-3-methoxyacrylat, Methyl-E-methoximino-[α-(2,5-dimethyloxy)-o-tolyl]acetamid.

Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)-pyrrol-3-carbonitril.

Zimtsäureamide wie 3-(4-chlorphenyl)-3-(3,4-dimethoxyphenyl)-acrylsäuremorpholid.

### Beispiel 1

### a) 2,6-Dichlor-5-fluorbenzamidoxim

In eine Suspension von 13,9 g 2,6-Dichlor-5-fluorbenzaldoxim und 90 ml Chloroform wurde bei Raumtemperatur so lange Chlor eingeleitet, bis das Ausgangsmaterial umgesetzt war. Nach Abfiltrieren des geringen ungelösten Rückstands und anschließendem Verdampfen des Lösungsmittels wurde das erhaltene gelbe Öl in 15 ml Tetrahydrofuran gelöst und bei -40 °C zu einer Lösung von 34 g Ammoniak in 100 ml Tetrahydrofuran zugetropft. Nach dem Auftauen und Eindampfen des Reaktionsgemischs wurde der Rückstand zwischen Methyltert.-butylether und 2 n Salzsäure verteilt. Die salzsaure Phase wurde auf einen pH-Wert von 7 eingestellt und mit Methyl-tert.-butylether extrahiert. Nach dem Verdampfen des Lösungsmittels konnten 5,8 g des gewünschten Produkts als Öl isoliert werden.
NMR(CDCl₃) in ppm: 4,85 s,br. (2H); 7,1-7,2 m(1H) ; 7,25-7,35 m (1H); 8,4 s,br. (1H)

### b) 2,6-Dichlor-5-fluorbenzamid-[O-cyclopropylmethyl]oxim

Zu einer Mischung von 3,8 g des unter a) erhaltenen Produkts und 2,5 g Cyclopropylmethylbromid in 30 ml Dimethylformamid wurden bei 0°C portionsweise innerhalb von 15 Minuten 0,54 g Natriumhydrid (80%ig) gegeben und 8 h bei 5°C nachgerührt. Der Ansatz wurde in Wasser eingerührt und dreimal mit je 70 ml Cyclohexan extrahiert. Nach dem Verdampfen des Cyclohexans wurden 3,6 g des gewünschten Produkts als Öl isoliert.
NMR(CDCl₃) in ppm: 0,25-0,35 m (2H); 0,4-0,50 m (2H); 1,1-1,25 m (1H); 3,9 d (2H); 4,8 s,br (2H); 7,1-7,2 m (1H); 7,25-7,35 m (1H)

### c) N-Phenylacetyl-2,6-dichlor-5-fluorbenzamid-[O-cyclopropylmethyl]oxim

1,15 g des nach b) erhaltenen Produkts und 0,9 g Phenylessigsäurechlorid wurden in 30 ml Toluol 15 h am Rückfluß erhitzt. Nach dem Abkühlen wurden 40 ml Wasser zugesetzt und auf pH 11 eingestellt. Aus der Toluolphase wurden nach Verdampfen des Lösungsmittels 1,5 g eines kristallinen Rohprodukts erhalten, aus dem nach Umkristallisieren 0,7 g des gewünschten Produkts mit einem Fp. von 71-72 °C erhalten wurden.
NMR(CDCl₃) in ppm: 0,15-0,25 m (2H); 0,45-0,55 m (2H); 1,1-1,15 m (1H); 3,65 s (2H); 3,85 d (2H); 7,05-7,15 m (1H); 7,2-7,4 m (6H); 8,5 s,br(1H).

Die auf diese Weise hergestellten Benzamidoxim-Derivate der Formel I sind der Tabelle 2 zu entnehmen.

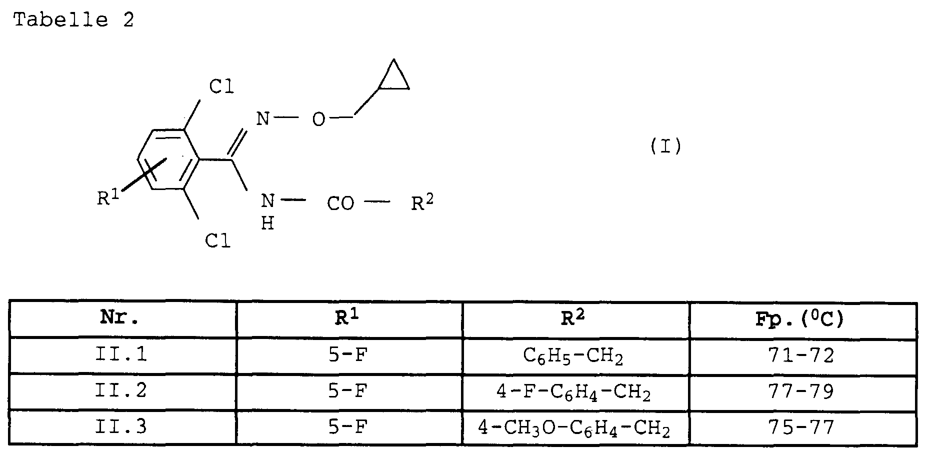

### Beispiel 2: Wirksamkeit gegen Weizenmehltau

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63% Cyclohexanon und 27 % Emulgiermittel angesetzt wurde, bis zur Tropfnässe besprüht und 24 h nach dem Antrocknen des Spritzbelages mit Sporen des Weizenmehltaus (Erysiphe graminis f.sp.tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung visuell in % der gesamten Blattfläche ermittelt.

Die mit den Wirkstoffen II.1 und II.3 der Tabelle 2 behandelten Pflanzen zeigten keinen Befall, während die unbehandelten Pflanzen zu 80 % befallen waren.

### Anwendungsbeispiel 1 - Wirksamkeit gegen Weizenmehltau

Blätter von in Töpfen gewachsenen Weizenkeimlingen der Sorte 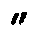 Kanzler" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Erysiphe graminis forma specialis tritici) bestäubt. Die Versuchspflanzen wurden anschließend im Gewächshaus bei Temperaturen zwischen 20 und 24°C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

| Wirkstoff | %-Befall der Blätter nach Applikation von ... ppm-haltiger wäßriger Wirkstoffaufbereitung | |
|---|---|---|
| | 63 | 16ppm |
| Tabelle 2. Nr. II.1 | 0 | 0 |
| Tabelle 2. Nr. II.2 | 5 | 5 |
| Tabelle 2. Nr. II.3 | 5 | 5 |
| Unbehandelt | 80 | |

## Patentansprüche

1. Benzamidoxim-Derivate der Formel I 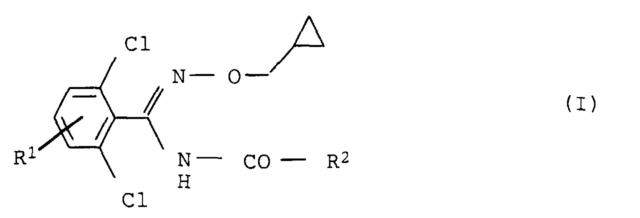 in der die Substituenten die folgende Bedeutung haben:
R¹ Fluor
R² Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches ggf. einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazol-C₁-C₄-Alkyl, welches ggf. einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann.

2. Benzamidoxim-Derivate der Formel I nach Anspruch 1, in der R² für Benzyl, welches am Phenylring einen bis drei Substituenten ausgewählt aus der Gruppe bestehend aus Halogen, C₁-C₄-Alkyl, C₁-C₄-Halogenalkyl,C₁-C₄-Alkoxy oder C₁-C₄-Halogenalkoxy tragen kann.

3. Benzamidoxime der Formel III

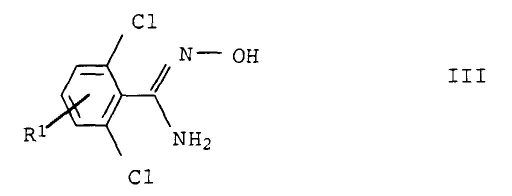

4. Benzamidoxime der Formel IV 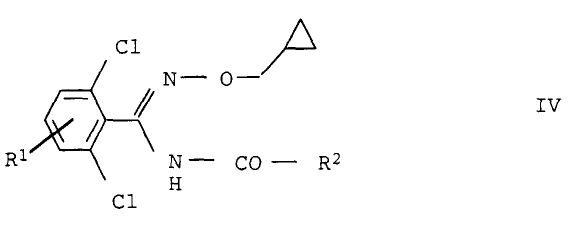 wobei R¹ und R² die in Anspruch 1 genannte Bedeutung haben.

5. Verwendung der Benzamidoxim-Derivate der Formel I gemäß Anspruch 1 zur Bekämpfung von Schadpilzen.

6. Verfahren zur Herstellung der Benzamidoxim-Derivate der Formel I gemäß Anspruch 1, dadurch gekennzeichnet, daß man Benzonitrile der Formel II mit Hydroxylamin oder dessen Salzen in wäßriger Lösung, vorzugsweise bei einem pH-Wert von größer 8 zu Benzamidoximen der Formel III umsetzt, diese anschließend mit einem Cyclopropylmethylhalogenid zu Benzamidoximen der Formel IV alkyliert und anschließend mit einem entsprechenden Säurehalogenid in Benzamidoxim-Derivate der Formel I überführt.

7. Fungizide Mittel, enthaltend eine fungizid wirksame Menge mindestens eines Benzamidoxim-Derivats der Formel I.

8. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer fungizid wirksamen Menge einer Verbindung der allgemeinen Formel I oder einem ein Benzamidoxim-Derivat der Formel I enthaltenden fungiziden Mittel gemäß Anspruch 7 behandelt.
